**Europäisches Patentamt**

**European Patent Office**

**Office européen des brevets**

(11) Numéro de publication : **0 379 394 B1**

(12) ## FASCICULE DE BREVET EUROPEEN

(45) Date de publication du fascicule du brevet :
**13.04.94 Bulletin 94/15**

(51) Int. Cl.$^5$ : **B01J 20/04,** C07C 7/12

(21) Numéro de dépôt : **90400029.6**

(22) Date de dépôt : **05.01.90**

(54) **Utilisation d'un adsorbant pour la purification de polyoléfines.**

(30) Priorité : **18.01.89 FR 8900530**

(43) Date de publication de la demande :
**25.07.90 Bulletin 90/30**

(45) Mention de la délivrance du brevet :
**13.04.94 Bulletin 94/15**

(84) Etats contractants désignés :
**AT BE CH DE DK ES FR GB GR IT LI LU NL SE**

(56) Documents cités :
**EP-A- 0 111 911**
**US-A- 4 433 981**
**US-A- 4 571 445**

(73) Titulaire : **RHONE-POULENC CHIMIE**
**25, quai Paul Doumer**
**F-92408 Courbevoie Cédex (FR)**

(72) Inventeur : **Garcin, Eric**
**72, rue Maurice Arnoux**
**F-92120 Montrouge (FR)**
Inventeur : **Cartier, Claude Bernard**
**56, rue Jean Jaurès**
**F-94190 Villeneuve Saint Georges (FR)**
Inventeur : **Quemere, Eric**
**11Bis, rue de Martray**
**F-95240 Cormeilles en Parisis (FR)**

(74) Mandataire : **Esson, Jean-Pierre et al**
**RHONE-POULENC CHIMIE Service Brevets**
**Chimie 25, quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne l'utilisation d'un adsorbant pour purifier des oléfines. Elle concerne plus particulièrement l'utilisation d'un adsorbant à base d'alumine pour la purification de polyoléfines obtenus par polymérisation d'oléfines en présence d'un système de catalyseurs de coordination, et plus précisément le milieu réactionnel obtenu dans ce procédé de polymérisation.

La polymérisation des oléfines est réalisée généralement, en présence de catalyseurs de polymérisation comprenant des éléments des groupes IV B, V B, VI B, du classement périodique des éléments et plus particulièrement, le vanadium, le titane et le zirconium. Ces catalyseurs comprennent également comme agents réducteurs des composés organométalliques (alkyl métallique), hydrures métalliques ou hydroxydes de métaux. Ces catalyseurs sont généralement appelés des catalyseurs de transition et présentent une grande activité catalytique pour la polymérisation d'oléfines.

Toutefois, les polyoléfines obtenues sont polluées par des résidus métalliques provenant de ces catalyseurs et il est donc indispensable de les épurer avant leur utilisation, pour éviter toute coloration, dégradation de celles-ci et diminuer leur toxicité.

En outre, les procédés de polymérisation des oléfines comprennent généralement une unité de récupération des solvants ou monomères contenus dans les polyoléfines, ces solvants et monomères étant recyclés dans l'unité de polymérisation. La présence de métaux dans ces composés engendre des problèmes de corrosion de l'installation.

Pour épurer ces oléfines, il est connu d'utiliser différents adsorbants, notamment des alumines. Par exemple, EP-A-0 237 175, décrit un procédé d'épuration d'oligomères d'oléfines en contactant le courant du produit de reaction avec des alumines, et/ou des alumines activées pour l'adsorption des impuretés contenant du phosphore.

Parmi les adsorbants utilisés, il est avantageux d'utiliser des adsorbants sous forme de billes qui peuvent être manipulées plus aisément que de la poudre ou des blocs de formes variées. En effet, un produit sous forme de billes peut être véhiculé tant pour le chargement que le déchargement des colonnes d'épuration par des systèmes pneumatiques par exemple.

Parmi les adsorbants, l'alumine peut être aisément conformée en billes. Toutefois, cette alumine intéressante pour sa facilité de manipulation et pour ses bonnes propriétés d'adsorption des métaux, présente un inconvénient majeur à savoir qu'elle favorise l'isomérisation des solvants contenu dans les polyoléfines, comme par exemple le butène -1. En conséquence, les solvants récupérés ne peuvent être totalement recyclés et doivent souvent être soumis à une épuration supplémentaire pour éliminer les produits isomérisés.

L'invention a notamment pour objet de remédier à ces inconvénients en proposant l'utilisation d'un adsorbant à base d'alumine présentant un faible pouvoir isomérisant et pouvant être facilement conformée en billes par exemple et plus généralement dans une forme permettant une manipulation aisée.

A cet effet, l'invention propose l'utilisation d'un adsorbant à base d'alumine comprenant au moins un composé d'un élément choisi dans le groupe des alcalins ou alcalino-terreux, dans une teneur comprise entre 15 mmole et 100 mmole d'élément par 100 g d'alumine, pour l'adsorption des éléments des groupes IVB, VB, VIB contenus dans un milieu réactionnel obtenu dans un procédé de polymérisation d'oléfines.

Sauf indication contraire, les concentrations sont exprimées en millimoles d'élément pour 100 g d'alumine calcinée à 300°C pendant 3 heures.

Cet adsorbant présente un pouvoir isomérisant des oléfines faible.

Le pouvoir isomérisant d'un adsorbant est déterminé par le test suivant :

On détermine le pouvoir isomérisant de ces produits en réalisant un test d'isomérisation du butène -1 en cis et trans butène -2.

Pour cela, on introduit 500 mg d'adsorbant broyé ( de particules comprises entre environ 400 et 500 $\mu$m) dans un réacteur. Le produit est conditionné pendant 2 heures à 250°C sous balayage d'hélium sous un débit de 2,5 l/h.

Puis le produit est porté à 400°C et on injecte 1 ml de butène dans le flux d'hélium.

L'analyse des gaz de sortie par chromatographie permet de mesurer la quantité de butène -1, et cis et trans butène -2 récupérée.

On détermine, par le calcul, la constante d'équilibre thermodynamique $K_{th}(T)$ théorique et par le résultat des mesures, la constante d'équilibre $K(T)$ réelle.

$$K_{th}(T) = \frac{[\text{cis butène} - 2]_e + [\text{trans–butène} - 2]_e}{[\text{butène} - 1]_e + [\text{cis butène} - 2]_e + [\text{trans} - \text{butène} - 2]_e}$$

$$K(T) = \frac{[\text{cis butène} - 2] + [\text{trans butène} - 2]}{[\text{butène} - 1] + [\text{cis butène} - 2] + [\text{trans butène} - 2]}$$

T est la température du butène à la sortie du réacteur les autres valeurs représentant les concentrations en sortie de réacteur ou à l'équilibre [ ]$_e$ pour la température T.

Le pouvoir isomérisant ou taux d'isomérisation A(T) est donné par la formule suivante :

$$A(T) \ = \ \frac{K(T)}{K_{th}(T)} \times 100$$

Selon une caractéristique de l'invention, le composé de l'élément est un oxyde, hydroxyde ou sel de celui-ci. Bien entendu, sans pour cela sortir du cadre de l'invention, on peut utiliser un mélange de composés.

Parmi les composés on peut citer à titre d'exemple, en plus des hydroxydes, les sulfates, nitrates, halogénures, acétates formiates, carbonates et plus généralement les sels d'acides carboxyliques, par exemple.

Les composés préférés de l'invention sont les hydroxydes et les chlorures. Ces derniers présentent un avantage économique important tout en procurant un adsorbant présentant un taux d'isomérisation remarquablement faible.

Il est également possible, d'utiliser un mélange d'éléments toutefois, la teneur totale en ces éléments devra, de préférence être comprise entre 15 mmole et 100 mmole par 100 g d'alumine.

Parmi les éléments convenables, on peut citer le sodium, le potassium et le calcium comme éléments préférés.

Toutefois, quand le potassium est choisi, la teneur en cet élément est de préférence comprise entre 15 mmole et 80 mmole.

Comme alumine convenable pour l'invention, on peut citer les alumines présentant une surface spécifique suffisante pour obtenir un taux d'adsorption acceptable des métaux. Typiquement, ces alumines ont une surface spécifique supérieure à 50 m²/g.

Ces alumines sont obtenues par des procédés classiques tels que le procédé par précipitation ou gel, et le procédé par déshydratation rapide d'un hydroxyde d'alumine.

Ces dernières alumines sont celles préférées par l'invention.

L'incorporation de l'élément ou des éléments dans l'alumine peut être réalisée par tous procédés, tel que coprécipitation de l'élément avec l'alumine ou imprégnation avant ou après mise en forme de l'alumine par une solution du ou des composés du ou des éléments.

Selon une caractéristique préférée de l'invention, le procédé de fabrication des adsorbants consiste à imprégner de l'alumine, de préférence sous forme de billes, par une solution aqueuse d'un sel ou d'un hydroxyde de l'élément à incorporer, puis à sécher l'alumine et, éventuellement, la soumettre à un traitement thermique pour stabiliser la surface spécifique de l'alumine.

Ce traitement thermique est réalisé à une température déterminée en fonction soit de la température d'utilisation de l'adsorbant soit de la surface spécifique désirée.

Il peut être également possible de faire subir un traitement thermique à une température plus élevée pour obtenir une dégradation thermique au moins partielle du composé, par exemple sous forme d'oxyde. Toutefois, cette dégradation n'est pas obligatoire et à titre d'exemple n'est pas nécessaire notamment quand on utilise des composés tels que des chlorures, nitrates, hydroxydes par exemple.

L'adsorbant de l'invention est notamment utilisé pour la purification de polyoléfines obtenues par polymérisation d'oléfines en présence de catalyseurs de coordination par adsorption des métaux provenant de ces catalyseurs. Ainsi, les oléfines encore contenues dans le mélange réactionnel sont purifiées sans subir d'isomérisation et donc peuvent être recyclées totalement.

Ce procédé permet également de purifier les polyoléfines.

L'invention sera illustrée par les exemples ci-dessous donnés uniquement à titre indicatif.

Les adsorbants indiqués ci-dessous sont fabriqués par traitement d'une alumine agglomérée en billes de diamètre 1,5 mm - 2,5 mm présentant une surface spécifique de 295 m²/g mesurée par la méthode BET, un volume poreux total de 0,5 cm³/g et dont la teneur résiduelle en sodium est comprise entre 0,2 et 0,4 % (en poids).

Ces billes d'alumine sont commercialisées par la Société Rhône-Poulenc sous la dénomination commerciale "A 1,5 - 2,5".

Ces billes d'alumine sont ensuite réactivées pendant 3 heures à 300°C sous air, puis imprégnées par une solution d'un sel de ou des éléments ou un composé tel qu'un hydroxyde de cet élément. Le volume et la concentration de cette solution sont déterminés pour obtenir la concentration désirée de l'élément dans l'alumine.

Les produits imprégnés sont ensuite séchés pendant 12 heures à 110°C sous air.

Leurs pouvoirs isomérisants sont déterminés au moyen du test décrit ci-dessus.

Les différents résultats et caractéristiques des adsorbants sont rassemblés dans le tableau ci-dessous :

| Ex | Elément X | Solution d'imprégnation | Concentration en X dans l'adsorbant mmole / 100gAl$_2$O$_3$ | Pouvoir isomérisant A % |
|---|---|---|---|---|
| 1(1) | - | - | 0 | 63 % |
| 2 | Na | NaOH | 25 | 14 % |
| 3 | Na | NaOH | 62 | 5 % |
| 4(1) | Na | NaOH | 250 | 54 % |
| 5 | K | KOH | 26 | 8 % |
| 6 | K | KOH | 68 | 14 % |
| 7 | K | KOH | 15 | 16 % |
| 8(2) | Ca | Ca(COOCH$_3$)$_2$ | 44 | 13 % |
| 9(2) | Ca | Ca(OOCCH$_3$)$_2$ | 18 | 21 % |
| 10(2) | Na | NaOOCCH$_3$ | 36 | 9 % |
| 11(2) | Na | CH$_3$COONa | 90 | 9 % |
| 12 | Na | NaCl | 65 | 1 % |
| 13(1) | Na | NaOH | 12 | 42 % |
| 14(1) | K | KOH | 130 | 77 % |
| 15(2) | Li | CH$_3$COOLi | 45 | 14 % |
| 16(2) | Sr | Sr(OOCCH$_3$)$_2$ | 65 | 36 % |
| 17(2) | Mg | Mg(OOCH$_3$)$_2$ | 65 | 36 % |
| 18 | Ca | CaCl$_2$ | 45 | 11 % |

(1) essais comparatifs

(2) calciné à 600°C pendant 3 heures après imprégnation

Les résultats montrent clairement qu'une alumine comprenant un élément tel que le sodium, le potassium, le calcium présente un pouvoir isomérisant très nettement inférieur à celui d'une alumine non traitée (exemple 1). De plus, ces exemples montrent également que la diminution du pouvoir isomérisant de l'alumine est obtenue pour un domaine de concentration déterminée.

Ainsi, les alumines non traitées qui contiennent du sodium comme impuretés ne peuvent convenir car la concentration en sodium est trop faible.

Il en est de même pour les alumines contenant un pourcentage élevé de sodium (5 % et plus) notamment utilisées dans l'adsorption d'impuretés acides.

Les alumines comprenant un élément comme le lithium, le strontium et le magnésium présentent également un pouvoir isomérisant nettement inférieur à celui d'une alumine non traitée (exemple 1).

## Revendications

1. Utilisation d'un adsorbant à base d'alumine comprenant au moins un composé d'un élément choisi dans le groupe formé par les alcalins et les alcalino-terreux, ledit élément étant présent dans une teneur comprise entre 15 mmole et 100 mmole pour 100 g d'alumine, pour l'adsorption des éléments des groupes IVB, VB, VIB contenus dans un milieu réactionnel obtenu dans un procédé de polymérisation d'oléfines.

2. Utilisation selon la revendication 1, caractérisée en ce que le composé de l'élément est un oxyde, hydroxyde ou sels dudit élément.

3. Utilisation selon la revendication 1 ou 2, caractérisée en ce que le composé de l'élément est un sulfate, nitrate, halogénure, acétate, formiate, carbonate ou sel d'acide carboxylique dudit élément.

4. Utilisation selon l'une des revendications 1 à 3, caractérisée en ce que le composé de l'élément est un chlorure ou un hydroxyde.

5. Utilisation selon l'une des revendications 1 à 4; caractérisée en ce que l'élément est choisi dans le groupe comprenant le sodium, le potassium, le calcium.

6. Utilisation selon la revendication 5, caractérisée en ce que le potassium est présent dans une teneur comprise entre 15 et 80 mmole par 100 g d'alumine.

7. Utilisation selon l'une des revendications précédentes, caractérisée en ce que l'alumine présente une surface spécifique supérieure à 50 m$^2$/g.

8. Utilisation selon l'une des revendications précédentes, caractérisée en ce que l'adsorbant est sous forme de billes.

9. Utilisation selon l'une des revendications précédentes, caractérisée en ce que l'alumine est obtenue par déshydratation rapide d'un hydroxyde d'aluminium.

10. Utilisation selon l'une des revendications précédentes, caractérisée en ce que ladite polymérisation est effectuée en présence d'un système de catalyseur de coordination.

11. Utilisation selon l'une des revendications précédentes, caractérisée en ce que ledit adsorbant est préparé par :
    - imprégnation de l'alumine par une solution d'un composé d'un élément ou d'un mélange de composés,
    - séchage de ladite alumine imprégnée,
    - traitement thermique de ladite alumine séchée pour stabiliser sa surface.

12. Utilisation selon la revendication 11, caractérisée en ce que l'imprégnation est réalisée avant mise en forme de l'alumine.

13. Utilisation selon la revendication 11, caractérisée en ce que l'imprégnation est réalisée après mise en forme de l'alumine.

## Patentansprüche

1. Verwendung eines Sorbentmittels auf Aluminiumoxid-Basis, umfassend mindestens eine Verbindung eines Elements, das aus der von den Alkali und Erdalkali gebildeten Gruppe ausgewählt ist, wobei das Element zu einem zwischen 15 mMol und 100 mMol pro 100 g Aluminiumoxid eingeschlossenen Gehalt vorliegt, zur Adsorption von Elementen der Gruppen IVB, VB, VIB, die in einem Reaktionsmedium enthalten sind, das in einem Verfahren zur Polymerisation von Olefinen erhalten wird.

**2.** Verwendung nach Anspruch 1, dadurch gekennzeichnet, daß die Verbindung des Elements ein Oxid, Hydroxid oder Salz des Elements ist.

**3.** Verwendung nach Anspruch 1 oder 2, dadurch gekennzeichnet, daß die Verbindung des Elements ein Sulfat, Nitrat, Halogenid, Acetat, Formiat, Carbonat oder Carbonsäuresalz des Elements ist.

**4.** Verwendung nach einem der Ansprüche 1 bis 3, dadurch gekennzeichnet, daß die Verbindung des Elements ein Chlorid oder Hydroxid ist.

**5.** Verwendung nach einem der Ansprüche 1 bis 4, dadurch gekennzeichnet, daß das Element aus der Natrium, Kalium, Calcium umfassenden Gruppe ausgewählt ist.

**6.** Verwendung nach Anspruch 5, dadurch gekennzeichnet, daß Kalium zu einem zwischen 15 und 80 mMol pro 100 g Aluminiumoxid eingeschlossenen Gehalt vorliegt.

**7.** Verwendung nach einem der vorangehenden Ansprüche, dadurch gekennzeichnet, daß das Aluminiumoxid eine Spezifische Oberfläche oberhalb von 50 m$^2$/g aufweist.

**8.** Verwendung nach einem der vorangehenden Ansprüche, dadurch gekennzeichnet, daß das Sorbentmittel in Form von Kugeln vorliegt.

**9.** Verwendung nach einem der vorangehenden Ansprüche, dadurch gekennzeichnet, daß das Aluminiumoxid durch schnelle Dehydratisierung eines Aluminiumhydroxids erhalten ist.

**10.** Verwendung nach einem der vorangehenden Ansprüche, dadurch gekennzeichnet, daß die Polymerisation in Gegenwart eines Koordinationskatalysator-Systems bewirkt wird.

**11.** Verwendung nach einem der vorangehenden Ansprüche, dadurch gekennzeichnet, daß das Sorbentmittel hergestellt ist durch:
- Imprägnieren von Aluminiumoxid mit einer Lösung einer Verbindung eines Elements oder einer Mischung von Verbindungen,
- Trocknen des imprägnierten Aluminiumoxids,
- thermisches Behandeln des getrockneten Aluminiumoxids, um seine Oberfläche zu stabilisieren.

**12.** Verwendung nach Anspruch 11, dadurch gekennzeichnet, daß die Imprägnierung vor der Formung des Aluminiumoxids durchgeführt wird.

**13.** Verwendung nach Anspruch 11, dadurch gekennzeichnet, daß die Imprägnierung nach der Formung des Aluminiumoxids durchgeführt wird.

## Claims

**1.** Use of an adsorbent with an alumina base, comprising at least one compound of an element selected from the group formed by alkali metals and alkaline earth metals, said element being present in an amount of between 15 mmole and 100 mmole for 100 g alumina, for the adsorption of group IVB, VB, VIB elements contained in a reaction medium obtained in a process for the polymerisation of olefins.

**2.** Use according to Claim 1, characterised in that the compound of the element is an oxide, a hydroxide or salts of said element.

**3.** Use according to Claim 1 or Claim 2, characterised in that the compound of the element is a sulphate, nitrate, halogenide, acetate, formate, carbonate or carboxylic acid salt of said element.

**4.** Use according to one of Claims 1 to 3, characterised in that the compound of the element is a chloride or a hydroxide.

**5.** Use according to one of Claims 1 to 4, characterised in that the element is selected from the group comprising sodium, potassium, calcium.

6. Use according to Claim 5, characterised in that the potassium is present in an amount of between 15 and 80 mmole per 100 g alumina.

7. Use according to one of the preceding claims, characterised in that the alumina has a specific surface area which is greater than 50 m$^2$/g.

8. Use according to one of the preceding claims, characterised in that the adsorbent is in the form of balls.

9. Use according to one of the preceding claims, characterised in that the alumina is obtained by rapid dehydration of an aluminium hydroxide.

10. Use according to one of the preceding claims, characterised in that said polymerisation operation is carried out in the presence of a coordination catalyst system.

11. Use according to one of the preceding claims, characterised in that said adsorbent is prepared by:
    - impregnation of the alumina by a solution of a compound of an element or of a mixture of compounds,
    - drying of said impregnated alumina,
    - heat treating said dried alumina to stabilise its surface area.

12. Use according to Claim 11, characterised in that the impregnation operation is carried out before the shaping of the alumina.

13. Use according to Claim 11, characterised in that the impregnation operation is carried out after the shaping of the alumina.